(19) Europäisches Patentamt
European Patent Office
Office européen des brevets

(11) Publication number: **0 002 304**
**B1**

(12) **EUROPEAN PATENT SPECIFICATION**

(45) Date of publication of patent specification: **29.09.82**

(51) Int. Cl.$^3$: **C 07 D 333/28**

(21) Application number: **78200331.3**

(22) Date of filing: **01.12.78**

(54) **Method of preparing a 3-iodothiophene.**

(30) Priority: **06.12.77 GB 5074977**

(43) Date of publication of application:
**13.06.79 Bulletin 79/12**

(45) Publication of the grant of the patent:
**29.09.82 Bulletin 82/39**

(84) Designated Contracting States:
**BE CH DE FR GB LU NL SE**

(56) References cited:
**Chemical Abstracts vol. 59, no. 12, 9 December 1963, Columbus, Ohio, U.S.A., S. GRONOWITZ et al "Iodo thiopenes and bithienyls" column 13920a**
**Chemical Abstracts vol. 60, no. 9, 27 April 1964, Columbus, Ohio, U.S.A., R. G. R. BACON et al "Metal ions and complexes in organic reactions I. Substitution reactions between aryl halides and cuprous salts in organic solvents" column 10495a**

(73) Proprietor: **Océ-Andeno B.V.**
**Grubbenvorsterweg 8**
**NL-5928 NX Venlo (NL)**

(72) Inventor: **Houbiers, Joannes Paulus Marie**
**Metternichstraat 11**
**Tegelen (NL)**
Inventor: **Thijssen, Francina Helena Johanna**
**Vastenavondkampstraat 24**
**Venlo (NL)**

(74) Representative: **Kremer, Robert A.M., Drs. et al,**
**Océ-Nederland B.V. Patents & Information**
**Postbus 101**
**NL-5900 MA Venlo (NL)**

(56) References cited:
**BEILSTEINS HANDBUCH DER ORGANISCHEN CHEMIE, 4. Auflage, 3. und 4. Ergänzungswerk, Band 17, 1. Teil, 1974, Springer-Verlag, Berlin, Heidelberg, New York, page 251.**
**HOUBEN-WEYL "Methoden der organischen Chemie", 4. Auflage 1960, Georg Thieme Verlag, Stuttgart, Band V/4, pages 595 to 597.**

**EP 0 002 304 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Method of preparing a 3-iodothiophene

The invention is directed to a method of preparing a 3-iodothiophene compound from a corresponding 3-bromothiophene compound.

The conversion of 3-bromothiophene into 3-iodothiophene is already known. 3-Bromothiophene can be prepared from 2,3,5-tribromothiophene according to Gronowitz and Raznikiewicz, Org. Syntheses, Coll. Vol. V (1973) 149. Conversion into 3-iodothiophene can be accomplished in the way described by Gronowitz on page 42 in "Advances in Heterocyclic Chemistry", Vol. 1, edited by A. R. Katritzky and published by Academic Press, New York (1963). According to this method through halogen-metal interconversion between 3-bromothiophene and n-butyllithium at −70°C 3-thienyllithium could be prepared, which at this temperature would be stable enough to be chosen as the reagent for the preparation of 3-substituted thiophenes such as 3-iodothiophene. The method suffers from several drawbacks as a consequence of which it is hardly applicable on plant scale but only on laboratory scale. One of these drawbacks is the low reaction temperature, which is self-evident. Another drawback is the use of butyllithium. Apart from its being expensive, the use of butyllithium necessitates to making all kinds of provisions, notably safety provisions, because it is quite a dangerous inflammable substance.

The object of the invention is to provide a simple method of preparing 3-iodothiophene that obviates the drawbacks mentioned above.

Another object of the invention is to provide a method by which not only 3-iodothiophene but also various alkyl-substituted 3-iodothiophenes can be synthesized in an easy way and with good yields.

These objects are met by a method of preparing a 3-iodothiophene compound from a corresponding 3-bromothiophene comprising reacting with copper (I) iodide in a polar aprotic solvent and at an elevated temperature a 3-bromothiophene of formula

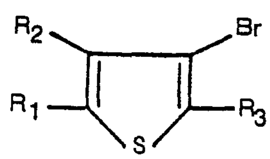

wherein

$R_1$ = H or a $C_{1-4}$ alkyl group
$R_2$ = H or a $C_{1-2}$ alkyl group
$R_3$ = H or a $C_{1-2}$ alkyl group with the proviso that $R_2$ and $R_3$ do not represent an alkyl group at the same time. Because of its easy nature and high yields the method is extremely suitable for the preparation of 3-iodothiophenes from a 3-bromothiophene of formula

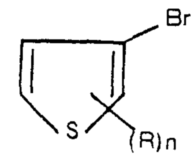

wherein

n = 0, 1 or 2 and R represents a $C_{1-2}$ alkyl group, and especially for the preparation of 3-iodothiophene from 3-bromothiophene. In the former case the two positions *ortho* to the Br atom, of course, may not be occupied by an alkyl group simultaneously as two ortho-alkyl groups may effect the reaction adversely.

It must be noted that transhalogenation of aromatic halides as such is not unknown. For example, in J. Chem. Soc. (1964) 1097 to 1107, Bacon and Hill describe substitutions of the type ArHal + Cux → ArX + CuHal, especially the conversion of 1-bromo- into 1-chloro-naphthalene, using cuprous salts in polar organic solvents and at temperatures of 110—140°C. However, their direct transhalogenation relates to the replacement of a halogen atom attached to an activated position by a lighter halogen atom.

The reverse reaction—replacement of a light halogen atom by a heavier one—did not succeed, or scarcely so, according to paragraph 2 of page 1101, for example. This failure is quite in accordance with what the handbooks in this field show: see, e.g., Houben-Weyl, 4. Auflage, Band V/4 page 596, paragraph 3 from bottom, as well as page 607/608 and Wagner: Synthetic Organic Chemistry 1965, pages 93 and 94. Thus, aryl halides do not permit direct replacement of a halogen atom by a heavier one, particularly not if the halogen atom does not occupy an activated position. The only alternative available is the roundabout route using a Grignard or lithium compound.

Therefore, it is surprising that 3-bromothiophene, even if it is substituted with one or more deactivating alkyl groups, permits direct replacement of the bromine atom by a iodine atom.

The amount of copper (I) iodide to be used may vary from a deficiency to an excess and, preferably, lies in the range of 0.5 to 2.5, especially 1.2 to 1.7 moles per mole of the 3-bromothiophene compound.

Examples of suitable polar aprotic solvents are dimethyl sulphoxide (DMSO), dimethylformamide (DMF), pyridines like 2-, 3- and 4-picoline, isoquinoline and hexamethyl phosphoric acid triamide (HMPA). Preferably DMSO or HMPA are used. More preferably, however quinoline is used because it is easily recuperated. Moreover, the highest yields are obtained in this solvent.

The reaction must be carried out at an elevated temperature, i.e. a temperature above

100°C, but preferably, a temperature between 120° and 160°C is chosen.

The 3-iodothiophene compounds obtained according to the method of the invention are useful intermediates, e.g. in organic chemistry research and for the preparation of a 3-thienylmalonic acid or ester thereof according to our co-pending U.K. Patent Application G.B. 2 009 158 A.

The following Examples will serve to illustrate the invention.

Example 1

A mixture of 326 g (= 2.0 moles) 3-bromothiophene and 571 g (= 3.0 moles) copper (I) iodide in 750 ml quinoline was stirred during a period of 12 hours at a temperature of 140°C. After the reaction mixture had been cooled down to 70°C 750 ml water and 750 ml concentrated HCl were added. The mixture was then steam distilled, and the distilled organic phase, after separation from the aqueous phase, washed with a solution of bicarbonate, dried and fractionated under reduced pressure. An amount of 126 g 3-iodothiophene was collected, which corresponds to a yield of 30%. The first runnings (238 g with a boiling range of 66°—87°C at 30 mmHg) contained, apart from non-reacted 3-bromothiophene, another 63.0 g (= 15%) 3-iodothiophene.

Example 2

A mixture of 97.8 g (= 0.6 moles) 3-bromothiophene and 171.4 g (= 0.9 moles) copper (I) iodide in 400 ml quinoline was stirred during a period of 20 hours at a temperature of 140°C. Then the heating and stirring was stopped. After the reaction mixture had been cooled down to 40°C 600 ml water and 400 ml concentrated HCl were added. The precipitated copper salts were collected on a Büchner funnel by filtration, crushed in a mortar and washed with petroleum-ether 40—60 (five times with 100 ml each). The aqueous phase was extracted twice with 100 ml petroleum-ether 40—60. All organic phases were joined and dried over magnesium sulphate. After removal of the petroleum-ether under reduced pressure the result was only 90 g of a product still containing petroleum-ether.

Therefore the aqueous layer and the copper salts were put together and this mixture steam distilled in a Dean-Stark apparatus. This resulted in 25 g of an oily product which were combined with the afore-mentioned 90 g.

Purification by distillation over a 99 cm vigreux column yielded:

1) a fraction of 54.2 g with a boiling range of 90°—95°C (120 mm Hg); $n_D^{20}$ = 1.5939. GLC analysis showed it to consist of 97% 3-bromothiophene and 3% 3-iodothiophene.

2) a fraction of 45.1 g with a boiling range of 100°—105°C (65 mm Hg); $n_D^{20}$ = 1.6572. GLC analysis showed it to consist of 1% 3-bromothiophene and 99% 3-iodothiophene.

3) a residue of 6 g, containing according to GLC analysis another 3.6 g 3-iodothiophene.

The quantity non-reacted 3-bromothiophene thus amounted to 53.1 g (= 0.326 moles or 54.3% calculated to the 3-bromothiophene started from). The demonstrated amount of 3-iodothiophene was 49.9 g (= 0.238 moles). This amounted to 39.7% calculated to the 3-bromothiophene started from or 87% calculated to the consumed 3-bromothiophene.

Example 3

This Example shows how the yield of 3-iodothiophene varies with the varying quantity of copper (I) iodide applied. 65.2 g (= 0.4 moles) 3-bromothiophene and a varying amount of copper (I) iodide were stirred during a period of 18 hours at a temperature of 140°C in 250 ml quinoline. After the temperature had dropped to 70°C 150 ml water and 250 ml concentrated HCl were added. Steam distillation resulted in an aqueous layer which was not analysed and an organic phase in which the quantities of 3-bromo- and 3-iodothiophene were determined by means of GLC analysis. The results were as mentioned in the Table on the following page.

Example 4

A mixture of 88.5 g (= 0.50 mole) 3-bromo-4-methylthiophene and 143.3 g (= 0.75 moles) copper (I) iodide in 188 ml quinoline was stirred during a period of 18 hours at a temperature of 140°C. After the reaction mixture had been cooled down to 70°C 188 ml water and 188 ml concentrated HCl were added. The resultant mixture was then steam distilled. The aqueous phase of the distillate was extracted with chloroform. The chloroform phase was joined with the organic phase and then dried over magnesium sulphate. After removal of the solvent under reduced pressure the residue (92.7 g) was distilled at 10 mm Hg. The first runnings being neglected a 25 g fraction was obtained which consisted of 3-iodo-4-methylthiophene (90%) and 3-bromo-4-methylthiophene (10%).

Example 5

A mixture of 16 g (= 0.084 moles) 3-bromo-2,5-dimethylthiophene and 34.3 g (= 0.18 moles) copper (I) iodide in 60 ml quinoline was stirred during a period of 17 hours at a temperature of 140°C. Then the reaction mixture was cooled to 80°C and 60 ml water and 60 ml concentrated HCl were added. The mixture was steam distilled in a Dean-Stark apparatus. The organic phase of the distillate was isolated and dried over magnesium sulphate. 21.5 g of a crude reaction product resulted which acording to GLC analysis contained 9.3 g 3-bromo-2,5-dimethylthiophene and 5.4 g (= 0.023 moles or 27%) 3-iodo-2,5-dimethylthiophene. A 99% sample obtained by distillation showed a $n_D^{20}$ = 1.6108.

TABLE

| Started from | | Results (the organic phase after steam distillation) | | | | | | |
|---|---|---|---|---|---|---|---|---|
| | | | 3-bromothiophene | | | 3-iodothiophene | | |
| moles 3-bromo-thiophene | moles CuI | weight of the organic phase in grams | grams | moles | percentage | grams | moles | percentage* |
| 0.40 | 0.04 | 64.4 | 54.7 | 0.34 | 84 | 4.6 | 0.022 | 5.5 |
| 0.40 | 0.08 | 65.6 | 50.6 | 0.31 | 78 | 9.5 | 0.045 | 11.3 |
| 0.40 | 0.16 | 67.5 | 50.9 | 0.31 | 78 | 15.8 | 0.075 | 19 |
| 0.40 | 0.28 | 68.7 | 42.0 | 0.26 | 64 | 22.8 | 0.11 | 27 |
| 0.40 | 0.40 | 70.6 | 39.0 | 0.24 | 60 | 30.0 | 0.14 | 36 |
| 0.40 | 0.52 | 71.4 | 36.1 | 0.22 | 55 | 33.8 | 0.16 | 40 |
| 0.40 | 0.60 | 71.2 | 35.5 | 0.22 | 54 | 34.9 | 0.17 | 42 |

* percentages are calculated to the amount of 3-bromothiophene started from

Example 6

A mixture of 43.8 g (= 0.2 moles) 3-bromo-2,5-diethylthiophene and 57.1 g (= 0.3 moles) copper (I) iodide in 100 ml quinoline was stirred during a period of 24 hours at a temperature of 140°C and then poured out into a mixture of 175 g melting ice, 175 ml concentrated HCl and 175 ml chloroform. The chloroform layer was separated, washed with an aqueous solution of sodium bicarbonate and dried over magnesium sulphate. After removal of the solvent the residue was fractionated by means of spinning band distillation. Apart from 49% unreacted 3-bromo-2,5-diethylthiophene (25.6% 3-iodo-2,5-diethylthiophene ($n_D^{20}$ = 1.5850) were obtained. If the yield were calculated to the amount of consumed 3-bromo-2,5-diethylthiophene it would rise to 50.2%.

## Claims

1. A method of preparing a 3-iodothiophene from a corresponding 3-bromothiophene characterized by reacting a 3-bromothiophene of formula

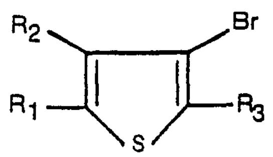

wherein

$R_1$ = H or a $C_{1-4}$ alkyl group

$R_2$ = H or a $C_{1-2}$ alkyl group

$R_3$ = H or a $C_{1-2}$ alkyl group with the proviso that $R_2$ and $R_3$ do not represent an alkyl group at the same time with copper (I) iodide in a polar aprotic solvent and at temperature above 100°C.

2. A method as claimed in claim 1 in which the 3-bromothiophene has the formula

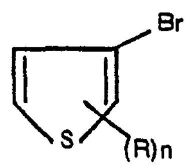

wherein

n = 0,1 or 2

R = a $C_{1-2}$ alkyl group with the proviso that both positions *ortho* to the Br atom do not simultaneously carry an alkyl group.

3. A method as claimed in claim 1 or 2 in which 0.5 to 2.5 moles of copper (I) iodide is used per mole of said 3-bromothiophene.

4. A method as claimed in claim 3 in which 1.2 to 1.7 moles of copper (I) iodide is used per mole of said 3-bromothiophene.

5. A method as claimed in any one of claims 1—4 in which the polar aprotic solvent is dimethyl sulphoxide, hexamethyl phosphoric acid triamide or quinoline.

6. A method as claimed in any one of claims 1—5 in which the reaction is carried out at a temperature between 120°C and 160°C.

## Revendications

1. Procédé de préparation d'un 3-iodothiophène à partir d'un 3-bromothiophène correspondant, caractérisé en ce qu'on fait réagir un 3-bromothiophène de formule

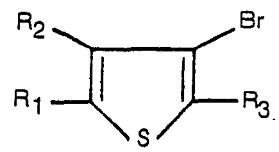

dans laquelle

$R_1$ est H ou un groupe alkyle en $C_{1-4}$

$R_2$ est H ou un groupe alkyle en $C_{1-2}$

$R_3$ est H ou un groupe alkyle en $C_{1-2}$ à condition que $R_2$ et $R_3$ ne représentent pas en même temps un groupe alkyle, avec de l'iodure de cuivre (I) dans und solvant aprotique polaire et à une température supérieure à 100°C.

2. Procédé selon la revendication 1, caractérisé en ce que le 3-bromothiophène répond à la formule

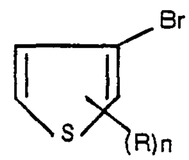

dans laquelle

n est 0,1 ou 2

R est un groupe alkyle en $C_{1-2}$ à condition que les deux positions ortho par rapport à l'atome de brome ne portent pas simultanément un groupe alkyle.

3. Procédé selon la revendication 1 ou 2, caractérisé en ce qu'on utilise de 0,5 à 2,5 moles d'iodure de cuivre (I) par mole du 3-bromothiophène mentionné.

4. Procédé selon la revendication 3, caractérisé en ce qu'on utilise de 1,2 à 1,7 mole d'iodure de cuivre (I) par mole du 3-bromothiophène mentionné.

5. Procédé selon l'une quelconque des revendications 1 à 4, caractérisé en ce que solvant aprotique polaire est le diméthyl sulfoxyde, le triamide de l'acide hexaméthyl phosphorique ou la quinoléine.

6. Procédé selon l'une quelconque des revendications 1 à 5, caractérisé en ce que la réaction est effectuée à une température comprise entre 120 et 160°C.

## Patentansprüche

1. Verfahren zur Herstellung eines 3-Jodthiophens aus einem entsprechenden 3-Bromthiophen, dadurch gekennzeichnet, dass man ein 3-Bromthiophen der Formel

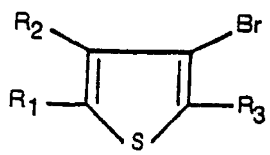

worin

$R_1$ = H oder eine Alkylgruppe mit 1—4 C-Atomen

$R_2$ = H oder eine Alkylgruppe mit 1—2 C-Atomen

$R_3$ = H oder eine Alkylgruppe mit 1—2 C-Atomen in einem polaren aprotischen Lösungsmittel und bei einer Temperatur über 100°C mit Kupfer (I)-jodid reagieren lässt, vorausgesetzt, dass $R_2$ und $R_3$ nicht gleichzeitig eine Alkylgruppe darstellen.

2. Verfahren nach Anspruch 1, dadurch gekennzeichnet, dass das 3-Bromthiophen die Formel

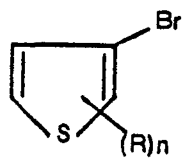

hat, worin

n = 0, 1 oder 2

R = eine Alkylgruppe mit 1—2 C-Atomen vorausgesetzt, dass beide Orthostellungen hinsichtlich des Bromatoms nicht gleichzeitig eine Alkylgruppe tragen.

3. Verfahren nach Anspruch 1 oder 2, dadurch gekennzeichnet, dass 0,5 bis 2,5 Mol Kupfer (I)-jodid pro Mol des verwendeten 3-Bromthiophens benutzt wird.

4. Verfahren nach Anspruch 3, dadurch gekennzeichnet, dass 1,2 bis 1,7 Mol Kupfer (I)-jodid pro Mol des verwendeten 3-Bromthiophens benutzt wird.

5. Verfahren nach Anspruch 1—4, dadurch gekennzeichnet, dass das polare aprotische Lösungsmittel Dimethylsulfoxid, Hexamethylphosphorsäuretriamid oder Chinolin ist.

6. Verfahren nach Anspruch 1—5, dadurch gekennzeichnet, dass die Reaktion bei einer Temperatur zwischen 120°C und 160°C durchgeführt wird.